# EUROPEAN PATENT SPECIFICATION

(11) **EP 2 124 058 B1**
(45) Date of publication and mention of the grant of the patent: **05.03.2014**
(21) Application number: 09168105.6
(22) Date of filing: 05.01.2007
(51) Int. Cl.: G01N 33/68

(54) **Acne diagnostic method by using lesions biomarkers and in vitro screening method for identifying modulators thereof**
Diagnostik von Akne mittels Biomarker für Verletzungen und Screenung von Modulatoren davon
Méthode de diagnostic basée sur des biomarqueurs de lésions acnéiques et procédé de criblage de leurs modulateurs

(30) Priority: 05.01.2006 US 756212 P
(43) Date of publication of application: 25.11.2009
(62) Divisional of application: 07712007.9
(73) Proprietor: Galderma Research & Development, 06410 Biot (FR)
(72) Inventor: Thiboutot, Diane, Hershey, PA PA 17033 (US)
(74) Representative: Starck-Loudes, Anne-Caroline

(56) References cited:
- WO-A-02/43758
- US-A1- 2003 211 105
- TRIVEDI NISHIT R ET AL: "Gene array expression profiling in acne lesions reveals marked upregulation of genes involved in inflammation and matrix remodeling" JOURNAL OF INVESTIGATIVE DERMATOLOGY, NATURE PUBLISHING GROUP, GB, vol. 126, no. 5, May 2006 (2006-05), pages 1071-1079, XP002417785 ISSN: 0022-202X
- GAO ZHONGLI ET AL: "Unraveling the chemistry of chemokine receptor ligands." CHEMICAL REVIEWS SEP 2003, vol. 103, no. 9, September 2003 (2003-09), pages 3733-3752, XP002545145 ISSN: 0009-2665
- JUGEAU S ET AL: "Induction of toll-like receptors by Propionibacterium acnes" BRITISH JOURNAL OF DERMATOLOGY, vol. 153, no. 6, December 2005 (2005-12), pages 1105-1113, XP002433923 ISSN: 0007-0963
- VOWELS B R ET AL: "Induction of proinflammatory cytokines by a soluble factor of Propionibacterium acnes: implications for chronic inflammatory acne." INFECTION AND IMMUNITY AUG 1995, vol. 63, no. 8, August 1995 (1995-08), pages 3158-3165, XP002545146 ISSN: 0019-9567
- BASAL E ET AL: "Antibody response to crude cell lysate of propionibacterium acnes and induction of pro-inflammatory cytokines in patients with acne and normal healthy subjects." JOURNAL OF MICROBIOLOGY (SEOUL, KOREA) JUN 2004, vol. 42, no. 2, June 2004 (2004-06), pages 117-125, XP002545147 ISSN: 1225-8873

## Description

The present invention relates to identification of acne lesions biomarkers/ genes expression products pattern and particularly inflammatory acne lesions biomarkers and their uses in acne diagnostic method and screening methods for identifying, modulators thereof and in vitro determination method of patient sensitivity to develop acne lesions.

Acne is the most common skin condition affecting millions of people worldwide. Patients with severe acne frequently face significant psychological and emotional problems due to the scarring associated with the disease. The pathogenesis of acne vulgaris is complex and incompletely understood.

The pathogenesis of acne has been linked to multiple factors such as increased sebum production, inflammation, follicular hyperkeratinization and the action of *Propionibacterium acnes* within the follicle.

Inflammation is a key component of the pathogenesis of acne. An immunological reaction to the gram-positive microbe *P. acnes* may play a major role in the initiation of the inflammatory reaction (De Young *et al,* 1984; Jappe *et al,* 2002). Recently published studies also implicate Toll Like receptor 2 (TLR-2) in inflammatory acne.

*Propionibacterium acnes* triggers pro-inflammatory cytokine release from inflammatory cells via activation of TLR-2, which in turn initiates an intracellular signaling cascade resulting in the transcription of genes such as interleukin-12 and interleukin-8 (Kim *et al,* 2002). Furthermore viable *P. acnes* and not heat-killed organisms, can stimulate the release of cytokines such as IL-1β, granulocyte/macrophage colony stimulating fraction (GM-CSF) and IL-8 (Nagy *et al*, 2005; Schaller *et al*, 2005).

While the exact initiating event causing acne still remains a mystery, there exists a debate as to whether hyperkeratinization of the follicular duct precedes the influx of inflammatory cells or vice versa. Recent studies support the later hypothesis by demonstrating that an increase in IL-1 activity occurs prior to the hyperproliferation around uninvolved follicles and this triggers the 'keratinocyte activation cycle'. (Freedberg *et al,* 2001; Jeremy *et al,* 2003)

Recent reports demonstrate that the skin expresses various antimicrobial peptides in response to the proliferation of pathogens as part of cutaneous innate immunity (Braff *et al*, 2005; Schroder, 2004; Selsted and Ouellette, 2005). Important amongst this group of anti-microbial agents include members of the human β defensin family and granulysin-derived peptides (Deng *et al*, 2005; Harder *et al*, 2004; Mclnturff *et al*, 2005). Human β defensin-1 and 2 (HBD-1 and HBD-2) are expressed in the pilosebaceous unit and their expression is upregulated in acne lesions (Chronnell *et al*, 2001). Recent studies have also discovered that select strains of *P*. *acnes* can activate HBD-2 through TLRs further confirming the importance of these peptides in inflammatory acne (Nagy, et al., 2005).

The document US2003211105 describes a method for identifying compounds inhibiting tumor growth and/or metastasis in a subject and method of treating cancer by using MCP1 or MCP2 receptor.

The document Gao et al, 2003, Chem Rev, 103, 3733-3752, 10 September 2009, describes a study on the chemistry of chemokines receptors and their ligands, and a method for identifying them.

The document WO02143758 describes a method of screening of candidate compounds for treating inflammatory skin diseases such as lupus erythematosus or atopic dermatitis and comprising the capacity determination of a compound to modulate expression of chemokines.

The document Jugeau et al, 2005, Brit J Derm, 153(6), 1105-1113, describes increased expression of TLR-2 and TLR-4 in acne lesions and establishes a link between several toll-like receptors and acne.

Acne research during the last 25 years has significantly increased our understanding about the etiological factors giving rise to acne. With the advent of gene array expression profiling however, new opportunities have arisen to re-examine the disease and potentially identify novel targets in its treatment.

Therefore there is a need to identify the specific genes expressed in inflammatory acne lesions compared to normal skin from acne patients and to test the hypothesis that differences in gene expression exist between normal skin from acne patients and skin from subjects without acne that may account for the predisposition to the disease and when acne is revealed to set up new therapeutics able to modulate/regulate genes expression.

In an attempt to understand the specific genes involved in inflammatory acne, the inventors of the present invention have performed gene expression profiling in acne patients. Skin biopsies were obtained from an inflammatory papule and from normal skin in 6 patients with acne. Biopsies were also taken from normal skin of 6 subjects without acne. Gene array expression profiling was conducted using Affymetrix U133A chips comparing lesional to non-lesional skin in acne patients and comparing non-lesional skin from acne patients to skin from normal subjects. Within the acne patients 211genes are upregulated in lesional skin compared to non-lesional skin. A significant proportion of these genes are involved in pathways that regulate inflammation and extra-cellular matrix remodeling and they include matrix metalloproteinase's 1 & 3, interleukin -8, human β defensin 4 and granzyme B. These data indicate a prominent role of matrix metalloproteinases, inflammatory cytokines and anti-microbial peptides in acne lesions. These studies are the first describing the comprehensive changes in gene expression in inflammatory acne lesions and as such may be of value in identifying potential therapeutic targets in inflammatory acne.

It is therefore proposed to target the genes as mentioned above and/or expression products of the genes to prevent and/or treat acne as well as acne associated disorders (e.g. hyperseborrhoea).

By acne it is understood, all acne forms especially simple acne, comedonic acne, papulopustular acne, papulocomedonic acne, nodulocystic acne, acne conglobata, cheloid acne of the nape of the neck, recurrent miliary acne, necrotic acne, neonatal acne, occupational acne, acne rosacea, senile acne, solar acne and medication-related acne.

In the context of the present invention « gene » refers to nucleic acid or nucleotide sequence encoding for a protein/biomarker expression. Said gene is also considered as a gene "target" for which a modulator is sought.
When the target is preferably a human gene or its expression product, the invention might use cells expressing the gene or the associated protein/biomarker by genomic incorporation or by transitory gene expression encoding protein.
Corresponding human sequences references are mentioned in tables 1 and 2.
Therefore, the present invention relates to acne lesions biomarkers and/or gene expression products as acne lesions biomarkers selected from the following list:
Matrix metalloproteinase 1 (MMP1) ; matrix metalloproteinase 3 (MMP3) ; interleukin 8 (IL8); beta 4defensin (DEFB4) ; skin-derived (SKALP) protease inhibitor 3 (PI3); chemokine (C-X-C motif) ligand 2 (CXCL2) ; apolipoprotein B mRNA editing enzyme (APOBEC3A) ; superoxide dismutase 2, mitochondrial (SOD2) ; granzyme B (GZMB) ; S100 calcium binding protein A9 (calgranulin B) (S100A9); chemokine (C-C motif) receptor 1 (CCR1); heparanase (HPSE); serum amyloid A2 (SAA2); leukotriene B4 receptor (LTB4R); tumor necrosis factor receptor superfamily member 1B (TNFRSF1B); complement component 3a receptor 1 (C3AR1); G protein-coupled receptor 65 (GPR65); baculoviral IAP repeat-containing 1 (BIRC1); CD14 antigen (CD14); serum/glucocorticoid regulated kinase (SGK); CD28 antigen (Tp44) (CD28); apoptosis caspase activation inhibitor (AVEN); TNF receptor-associated factor 3 (TRAF3); aldo-keto reductase family 1, member B10 (AKR1B10); phospholipase A2, group IIA (platelets, synovial fluid) (PLA2G2A); UDP glycosyltransferase 1 family, polypeptide A10 (UGT1A10); phospholipid scramblase 1 (PLSCR1); serum amyloid A2 (SAA2) ; fatty acid binding protein 5 (psoriasis-associated) (FABP5); arachidonate 5-lipoxygenase (ALOX5); phosphoinositide-3-kinase, catalytic, delta polypeptide (PIK3CD); secretoglobin, family 1 D, member 2 (SCGB1 D2); secretoglobin, family 2A, member 1 (SCGB2A1); transmembrane 4 superfamily member 3 (TM4SF3); mutS homolog 5 (E. coli) (MSH5); secretoglobin, family 2A, member 2 (SCGB2A2); mutS homolog 5 (E. coli) (MSH5); frizzled-related protein (FRZB); hypothetical protein MGC11242 (MGC11242); SRY (sex determining region Y)-box 10 (SOX10); keratin 18 (KRT18); lipase hepatic (LIPC); coagulation factor X (F10); hypothetical protein FLJ20280 (FLJ20280); fast troponin C2,(TNNC2); KDEL endoplasmic reticulum protein retention receptor 3 (KDELR3); KIAA0514; DKFZP564O243 protein; nuclear receptor subfamily 1, group D, member 1 (NR1D1); CD47 antigen (Rh-related antigen, integrin- associated signal transducer) (CD47); Thy1 cell surface antigen (Thy1); selectin P ligand (SELPG); TIMP metallopeptidase inhibitor 1(TIMP1); chemokine (C-C motif) ligand 21(CCL21); S100 calcium binding protein A9 (calgranulin B) (S100A9); chemokine (C-C motif) receptor 2 /// chemokine (C-C motif) receptor 2 (CCR2); chemokine (C-C motif) receptor 5 (CCR5); selectin L(lymphocyte adhesion molecule 1)(SELL).
Particularly, these biomarkers are inflammatory acne lesions biomarkers.

In the context of the invention, it is understood as biomarker a characteristic that is objectively measured and evaluated as an indicator of normal biologic processes, pathogenic processes, or pharmacologic responses to a therapeutic intervention (NIH definition).
Therefore, biomarkers are used to indicate or measure a biological process (for instance, levels of a specific protein in blood or fluids, genetic mutations, or abnormalities observed in tests). Detecting biomarkers specific to a disease can aid in the identification, diagnosis, and treatment of affected individuals and people who may be at risk but do not yet exhibit symptoms.

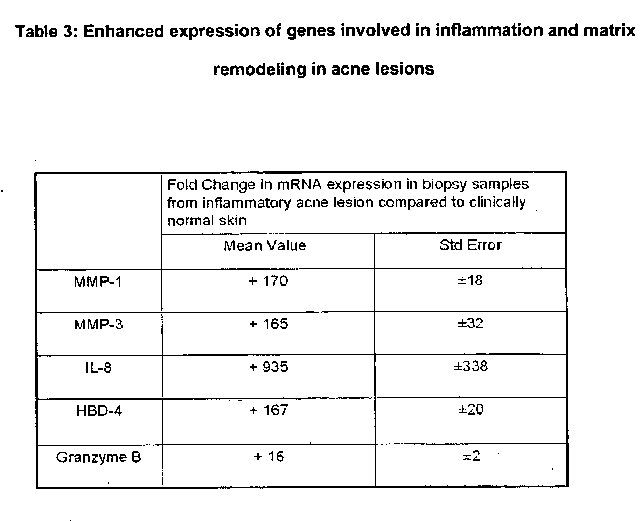

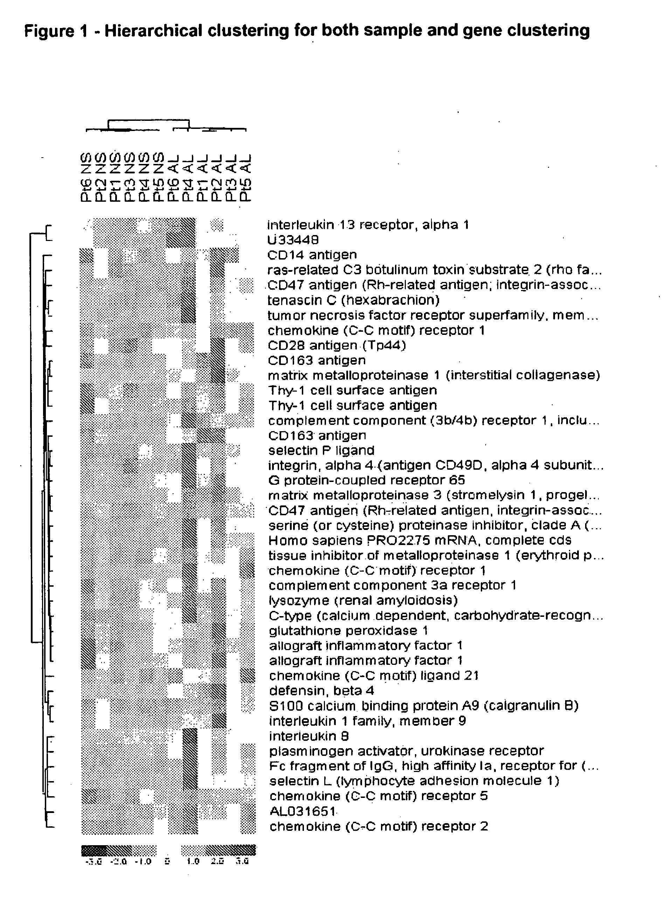

In addition, the present invention relates to the said gene expression product as "biological targets". By target it is understood an enzyme, a receptor, other protein or mRNA that can be modified by an external stimulus. The definition is context-dependent and can refer to the biological target of a pharmacologically active drug compound, or the receptor target of a hormone. The implication is that a molecule is "hit" by a signal/stimulus and its behavior is thereby changed.
In the context of invention, target of interest are those disclosed in tables 1 and 2, and figure 1 and more specifically above mentioned expression products.

### Diagnostic methods

One embodiment of the present invention encompasses an acne diagnostic method or an acne disease or hyperseborrehoea evolution follow-up method by using expression of genes or biomarkers/ gene expression products selected from the following list: receptor 1 chemokine (C-C motif) (CCR1); chemokine (C-C motif) receptor 2 /// chemokine (C-C motif) receptor 2 (CCR2); chemokine (C-C motif) receptor 5 (CCR5), said method comprises the step of comparing expression of said genes or genes expression products activity, in a patient biological sample with a subject "control" biological sample.
Gene expression products/ Biomarkers (e.g. Proteins) might be determined by any appropriate methods such as western-blot, IHC, MAS spectrometry analysis (MAldi-TOF and LC/MS analysis), Radioimmunoassay (RIA), Elisa or by any other methods well known by skilled in the art or by mRNA dosage by any appropriate methods well known by skilled in the art.
It is understood by subject "control" is a subject in healthy conditions or in non involved skin of acne conditions.

In the context of an acne disease or acne associated disorders evolution follow-up method, the « control » subject is understood as the same subject where biological sample was taken at a different time and preferentially at the beginning of the treatment or before the treatment (Baseline). The comparison of gene expression or Biomarkers/gene expression products levels, represents a tool to determine the product efficacy and decide whether or not continue the treatment with the same product.

Another embodiment of the invention is an *in vitro* determination method of patient sensitivity to develop acne lesions and/or hyperseborrhoea, which comprises the step of comparing genes expression or gene expression products levels, or activity of biomarkers as selected from the following list:
chemokine (C-C motif) receptor 1 (CCR1); chemokine (C-C motif) receptor 2 /// chemokine (C-C motif) receptor 2 (CCR2); chemokine (C-C motif) receptor 5 (CCR5), in a patient biological sample with a subject "control" biological sample.
Again, biomarkers and/or gene expression products levels might be measured by any appropriate methods such as western-blot, IHC, MAS spectrometry analysis (MAldi-TOF and LC/MS analysis), Radioimmunoassay (RIA), Elisa or by any other methods well known by skilled in the art and for example by ELISA dosage or by mRNA dosage by any appropriate methods well known by skilled in the art.
In this case, patient is asymptomatic and presents any acne visible symptoms. Therefore the «control» subject is one from healthy reference population.

In the context of above mentioned diagnostic methods, the biological sample might be any biological fluid sample (sebum, blood, urine, plasma....) or any sample extract by biopsy and is preferentially a skin sample.

### Screening methods

Another embodiment of the present invention is an in vitro screening method of drug candidates (or family lead compound) susceptible of preventing and/or treating acne as well as acne associated disorders (e.g. hypserborrhoea) comprising determining the capacity of said candidate to inhibit or down regulate expression of a selected gene or activity of said selected gene expression product selected from the following list: receptor 1 chemokine (C-C motif) (CCR1); chemokine (C-C motif) receptor 2 /// chemokine (C-C motif) receptor 2 (CCR2); chemokine (C-C motif) receptor 5 (CCR5). The said method comprises the step of determining the drug candidate capacity to modulate (e. g. down regulated or up regulate) said genes expression and/or said biomarkers/gene expression products levels mentioned in table 3 and/or their activities.
In a specific embodiment, the invention is an in vitro screening method of drug candidates susceptible of preventing and/or treating acne as well as acne associated disorders (e.g. hyperseborrhoea); said method comprising the following steps:
a. Collecting at least two biological samples : one mimics pathological acne lesion condition and the other mimics healthy condition;
b. Contacting at least one sample or a mixture of samples with one or more drug candidates to be tested;
c. Measuring gene expression or gene expression product level or activity in the biological samples or mixture obtained in b); the said gene expression product is selected from the following list: receptor 1 chemokine (C-C motif) (CCR1); chemokine (C-C motif) receptor 2 /// chemokine (C-C motif) receptor 2 (CCR2); chemokine (C-C motif) receptor 5 (CCR5);
d. Selecting drug candidates which are capable of modulating gene expression or gene expression product level or activity measured in said samples or mixture obtained in b) and comparing the levels with a control sample, not mixed with drug candidate.

By "modulate" it is understood any effect on expression or activity of biomarkers/gene expression products, any effect on genes or on activity of at least one of their expression promoter(s) and preferentially any effect inducing e. g. a down regulation or an up regulation, a stimulation, an inhibition, totally or partially.

In the context of the present invention and without particular notice, it is understood that «expression of biomarkers/gene expression product » refers to a quantity of a protein or any else product resulting from the transcription and/or translation of a gene. By activity it is meant biological activity.

By "activity of gene promoter(s)" it is meant the promoter(s) capacity to trigger DNA sequence transcription under the control of said promoter(s).

In a particular embodiment, in the screening method the gene expression products at step c) are selected from the following list or those mentioned in table 4:
Beta 4 defensin (DEFB4); skin-derived (SKALP) protease inhibitor 3 (PI3); ligand 2 chemokine (C-X-C motif) (CXCL2); apolipoprotein B mRNA editing enzyme (APOBEC3A); mitochondrial superoxide dismutase 2 (SOD2); granzyme B (GZMB) ; ligand 2 chemokine (C-X-C motif) (CXCL2); receptor 1 chemokine (C-C motif)(CCR1); leukotriene B4 receptor (LTB4R); tumor necrosis factor receptor superfamily member 1B(TNFRSF1B); complement component 3a receptor 1 (C3AR1); G protein-coupled receptor 65 (GPR65); baculoviral IAP repeat-containing 1(BIRC1); serum/glucocorticoid regulated kinase (SGK); CD28 antigen (CD28); apoptosis caspase activation inhibitor (AVEN); TNF receptor-associated factor 3 (TRAF3); aldo-keto reductase family 1, member B10 (AKR1B10); phospholipase A2, group IIA (platelets, synovial fluid)(PLA2G2A); UDP glycosyltransferase 1 family, polypeptide A10 (UGT1A10); phospholipid scramblase 1(PLSCR1); serum amyloid A2 (SAA2); fatty acid binding protein 5 (psoriasis-associated) (FABP5); phosphoinositide-3-kinase, catalytic delta polypeptide (PIK3CD); secretoglobin, family 1D, member 2 (SCGB1D2); secretoglobin, family 2A, member 1(SCGB2A1); transmembrane 4 superfamily member 3 (TM4SF3); mutS homolog 5 (MSH5); secretoglobin, family 2A, member 2 (SCGB2A2); frizzled-related protein (FRZB); SRY (sex determining region Y)-box 10 (SOX10); keratin 18 (KRT18); lipase hepatic (LIPC); coagulation factor X (F10); fast troponin C2 (TNNC2); KDEL endoplasmic reticulum protein retention receptor 3 (KDELR3); nuclear receptor subfamily 1, group D, member 1 (NR1D1); CD47 antigen (Rh-related antigen, integrin- associated signal transducer) (CD47); Thy1 cell surface antigen (Thy1); selectin P ligand (SELPG); TIMP metallopeptidase inhibitor 1(TIMP1); chemokine (C-C motif) ligand 21(CCL21); S100 calcium binding protein A9 (calgranulin B) (S100A9); chemokine (C-C motif) receptor 2 /// chemokine (C-C motif) receptor 2 (CCR2); chemokine (C-C motif) receptor 5 (CCR5); selectin L (lymphocyte adhesion molecule 1)(SELL).

The compounds to be tested are any kind of compounds, from natural or synthetic source. As synthetic compounds they might be chemically synthesized or from chemical compound data bank, with a defined structure or non characterized or present in a mixture of compounds.
Several technical assays are available for assessing compounds activity modulating above mentioned biomarkers/gene expression products.

According to a first embodiment, biological samples are transfected cells containing reporter gene operably under the control of a promoter (totally or partially) controlling the expression of an above mentioned gene. Therefore step c) above consists to measure the expression of the reporter gene.
The reporter gene may encode an enzyme that with its corresponding substrate, provides coloured product(s) such as CAT (chloramphenicol acetyltransferase), GAL (beta galactosidase), or GUS (beta glucuronidase). It might be either luciferase or GFP (Green Fluorescent Protein) gene.
Reporter gene protein dosage or its activity is typically assessed by colouring, fluorometric or chemoluminescence methods.

According to a second embodiment of the invention, biological samples are cells expressing the gene of interest and the step c) above consists to measure the activity of the gene product.

Any kind of cell is suitable for the invention. Cells may endogenously express the said gene like sebocyte. Organs may be suitable for the instant invention, from animal or human origin like preputial gland or sebaceous gland.
Transformed cells by heterologous nucleic acid encoding the gene expression product of interest might either be suitable. Preferably the said nucleic acid is from animal (preferred mammal) or human origin. A large variety of host cells is suitable for the invention and in particular Cos-7, CHO, BHK, 3T3, HEK293 cells. Cells are transiently or permanently transfected by a nucleic acid of interest with a well known by skilled in the art method and for instance calcium phosphate precipitation, DEAE-dextran, liposome, virus, electroporation or microinjection.

In the above described methods, expression levels of a gene of interest or reporter gene are determined according to transcription or translation rates.
By transcription rate it is understood, mRNA levels. By translation it is meant, protein production rate.
Quantitative or semi-quantitative methods for mRNA of gene of interest detection are well known by one skilled in the art.
Methods based on mRNA hybridation with nucleic probes are typically known (Northern Blot, RT-PCR, RNase protection). It might be advantageous to use detection markers such as fluorescent, radio-labelled, enzymatic agents or other ligands (for example avidine/biotine).

Gene translation rate may also be assessed by immunological assays of gene expression product. To this aim, polyclonal or monoclonal antibodies may be used. Antibodies manufacturing methods are well known by one skilled in the art. For instance, monoclonal antibody might be produced according to Kôhler and Milstein method (Nature (London), 256: 495- 497 (1975) or by cloning a nucleic acid expression clone in hybridoma.

Immunological dosages are assessed by solid or homogeny phase, in one or two time frames; with the so-called sandwich method or with competition method.
According to a preferred embodiment of the invention, the antibody is captured on or into solid support such as microplaques, polystyrene plaques or balls or paramagnetic balls. ELISA dosage, radio-immuno assays or other type of detecting methods may convey to detect antibody/antigen complex.

### Modulators

The term "inhibit" refers to a compound that reduces or decrease, restraint, down-regulate, prevent (totally or partially) or suppress, antagonise, stop, block biomarkers/gene expression product activity. By partially, it is meant a reduction of activity of at least 25%, preferred of at least 35%, more preferred of at least 50% and preferentially of at least between 70% and 90%.
Particularly, the modulator might interact and block the active site of the gene expression product like competitive inhibitor.
A preferred inhibitor is active in a solution at a concentration of at least less than 1µM, preferred less than 0,1 µM, preferentially less 0,01 µM.

The modulator might be an anti body and preferably a monoclonal antibody. Advantageously, the monoclonal antibody is administered to a patient in a sufficient quantity so as the measure a plasmatic concentration from about 0.01 µg/ml to about 100µg/ml, preferred from about 1 µg/ml to about 5µg/ml.

Modulator might be either a polypeptide, a DNA or RNA antisens, a si-RNA or a PNA ("Peptide nucleic acid", i-e with a polypeptidic chain substituted by purine and pyrimidine bases and having a DNA -like structure for hybridization to this latter)

In the in vitro screening method of drug candidate, selected drug candidates or modulator compounds are formulated into a composition, associated with a pharmaceutically acceptable vehicle. Those compositions are administered by oral, enteral, parenteral or topic route. Preferably, the route of administration is topic.
For oral route, the composition could be in a tablet form, pills, dragees, syrup, suspension, solution, powder, granules, emulsion, microspheres or nanospheres suspensions or lipid or polymeric vesicles compatible with a control release.
For parenteral route, composition could be in a solution or suspension form for injection or perfusion.
For topical route, the composition is particularly usable for the treatment of skin and mucosa and could be in a form of unguents, creams, milks, ointments, powders, tampons imbibes, solutions, gels, gel-cream, sprays, lotion, emulsions, suspensions or microspheres or nanospheres suspensions or lipid or polymeric vesicles compatible with a control release. Composition might either be in an anhydic or aqueous form or be an emulsion. In a preferred embodiment, composition is in a gel, cream or lotion form. Composition comprise from 0,001 to 10 %, preferred from 0,01 à 5 % by weight/total composition weight of modulator compound.

Composition may also comprise inert additives or mixture of them, such as
- wetting agents ;
- taste ameliorating agents ;
- preservative agents such as parahydroxybenzoïc acid esters;
- stabilizating agents;
- humidity regulating agents;
- pH regulating agents;
- osmotic pressure modulating agents;
- emulsifying agents ;
- UV-A et UV-B filters
- antioxydant agents such as alpha-tocopherol, butylhydroxyanisole or butylhydroxytoluene, Super Oxyde Dismutase, Ubiquinol or metal chelating agent.

The present invention will now be illustrated by means of the following figures and examples:

### Table 3 and Figure Legends

**Table 3 - Enhanced expression of genes involved in inflammation and matrix remodeling in acne lesions**
   To validate the micro-array findings we quantified the mRNA levels of 5 genes involved in inflammation using quantitative real time PCR. Table 3 describes the fold changes in mRNA expression for MMP-1, MMP-3, IL-8, HBD-4 and granzyme B respectively in inflammatory acne lesions and biopsies of normal skin from the same group of patients. Values shown here are means with standard errors indicated by the bars.
**Figure 1** **-Hierarchical clustering of genes involved in inflammation**
   Hierarchical clustering of genes involved in inflammation from 6 patients with skin biopsies taken at the site of the acne lesion (AL) and at a corresponding site of normal skin (NS) from the same patient. Each row represents a gene labeled with gene name or accession number and each column represents the patient sample. The color in each cell reflects the level of expression of the corresponding gene in the corresponding sample, relative to its mean level of expression in the entire set of biopsy samples. Expression levels greater than the mean are shaded in red and those below the mean are shaded in blue.
**Figure 2** **-Confirming changes in gene expression pattern by immunohistochemistry**
   Immunohistochemistry staining was performed on sections of acne skin and compared to those of clinically normal skin.
**Figure 3** **- Immunohistochemistry staining of MMP-1 in clinically normal skin and inflammatory acne.**
   MMP-1 expressed in the epidermis and sebaceous glands in inflammatory acne shown in (**b**) compared to IgG1 control shown in (**a**)**.** Variable levels of MMP-1 staining can be seen in serial sections of clinically normal skin from the same acne patient (**c**-**e**). A higher expression level of MMP-1 staining is seen in the epidermis in sections close to microscopically visible perifollicular inflammation (**c**) and progressively lower levels of MMP-1 staining is seen in sites distal from it (**d**, **e**).

### Example 1: Expression of inflammatory mediators, antimicrobial peptides and matrix metalloproteinases is increased in acne lesions compared to uninvolved skin

### Materials

The gene chips 'HG-U133A 2.0' were purchased from Affymetrix (Santa Clara, California). The MMP-1, MMP-3, IL-8, Human B-defensin 4 (HBD-4) and granzyme B primers for Real Time PCR were obtained from Applied Biosystems (California). The primary antibodies for immunohistochemistry for MMP-1 and IL-8 were purchased from R&D systems (Minneapolis, MN) and HBD-4 from Abcam Inc. (Cambridge, MA)

### Patient selection and tissue biopsies

Twelve patients including male and females ages 18 to 45 years were enrolled in the study, 6 patients with acne lesions on the back and 6 subjects without acne. The inclusion criteria for the acne lesion group included: a) males and females ages 18 to 45 years with inflammatory acne on their back b) subjects without other skin disease in the biopsy area c) subjects who were willing to have skin biopsies performed from their back and d) subjects that have not been treated with isotretinoin for acne within the previous 6 months.

The inclusion criteria for subjects without acne included: a) Males and females ages 18 to 45 years who were willing to have a skin biopsy performed from their back and b) subjects without other skin disease in the biopsy areas. The exclusion criteria (all subjects) included: subjects that were taking oral medications that might influence gene expression in the skin or applying topical medications in the target areas on the back. A punch biopsy (5mm) of the skin was performed at 2 sites on the back of acne patients, one at the site of inflammatory acne papule and other from a region of clinically normal skin. Normal subjects without acne were subjected to only one biopsy taken from the normal skin on the back.

### Extraction of RNA, labeling and hybridization to probe arrays

Skin samples were flash frozen and individually cryosectioned to facilitate RNA isolation. Total RNA was isolated from skin and DNase treated using the RNeasy Fibrous Tissue Kit (Qiagen Inc.,Valencia, CA) according to the manufacturer's instructions. RNA was ethanol precipitated to concentrate the sample and then quantified using a spectrophotometer. Approximately 2µg of total RNA from each sample was used to generate double stranded cDNA using a T7-oligo (dT) primer. Biotinylated cRNA, produced through in-vitro transcription, was fragmented and hybridized to an Affymetrix human U133A 2.0 microarray. The arrays were processed on a GeneChip Fluidics Station 450 and scanned on an Affymetrix GeneChip Scanner.

### Quantitative Real-Time PCR.

Quantitative real-time PCR was performed to confirm changes in the level of select genes from the array data. Complimentary DNA was generated from 1µg of total RNA, primed with oligo dT, using the Superscript First-Strand Synthesis System for RT-PCR (Invitrogen, Carlsbad, CA). Assays-on-Demand Taqman Universal PCR Master Mix and primer probe sets (Applied Biosystems) were used to run real-time PCR on ABI's 7900HT Fast Real-Time PCR System with 384-well plate block module (Applied Biosystems, Foster City, CA). Samples corresponding to 80ng total RNA input were run in triplicate for the reference gene TBP as well as 6 genes of interest (MMP-1, MMP-3, DEFB4, IL8, GZMB, and GATA6). No template and no RT controls were also run.

### Immunohistochemistry

In order to verify changes in gene expression at the protein level, a cohort of 4 additional subjects with acne were recruited to undergo biopsies of an inflammatory acne lesion and of uninvolved skin from the back. In addition, skin samples from additional subjects without acne were obtained to further assess the expression of the proteins of interest. Immunohistochemistry was performed on these samples following formalin fixation, paraffin embedding and tissue processing as previously described (). Sections were incubated with monoclonal antibody to MMP-1 (1:200) and beta-defensin 4 (1:100) and polyclonal antibody to IL-8 (1:50) overnight at 4°C. MMP-1 and IL-8 primary antibodies were purchased from R&D systems while HBD-4 was procured from Abcam. Slides were incubated with biotinylated secondary antibodies against the respective primary antibodies at a dilution of 1:500 for 60min at room temperature followed by 30-minute incubation with the ABC reagent (Vector Labs). The AEC kit from Vector labs was used as the chromogen, which stains as a red color. The sections were counterstained with hematoxylin and slides analyzed by microscopy.

### Clustering

Out of the 211 genes that were upregulated in acne lesions, 41 were identified as part of the inflammatory probe set generated from the NetAffix analysis center from the Affymetrix website (give web address). Hierarchical clustering of patient samples and genes involved in inflammation was performed using the Computer software dChip (Li and Wong. 2003) version 1.3. Patient samples included skin biopsies taken at the site of the acne lesion (AL) and at a site of clinically normal skin (NS) from the same patient. Normalized chip intensity data were imported into dChip. Gene information file for Affymetrix human genome HG-U133A array was obtained from dChip's website at www.dChip.org.

### Statistical Analyses

Before testing significant changes in gene expression, the expression signals were normalized by using the R-Affy package from Bioconductor (version 1.1, Irizarry et al. 2003a) to remove background noise and non-biological variations among arrays. The background noise was removed from the PM probe intensities using the "RMA" method (Irizarry et al. 2003b), which assumes a global model for the distribution of probe intensities and models the PM probe intensities as the sum of a normal noise component and an exponential signal component. Normalization was done using the quantile normalization method (Bolstad et al. 2003). Quantile normalization assumes that the expression of majority of genes on the arrays does not change in different treatments and the distribution of probe intensities for each array in the dataset is same. To remove outlier probes and summarize probe intensities within one probe set into a single expression value, "Tukey Biweight" method was applied to the background adjusted, normalized PM intensities. Expression values were obtained based on PM intensities not PM-MM intensities, because PM and MM intensities were found to be highly correlated which suggested that MM intensities composed of background noise as well as probe specific signals. Significant gene expression alterations were identified using computer software Significance Analysis of Microarrays (SAM) (Tusher et al. 2001). SAM assigns a score to each gene on the basis of gene expression change relative to the standard deviation of repeated measurements and identifies genes with statistically significant changes in expression using a permutation procedure. SAM controls the false positives resulting from multiple comparisons through controlling the false discovery rate (FDR) (Benjamini and Hochberg, 1995). FDR is defined as the proportion of false positive genes among all genes that are considered significant.

### Results

A distinct pattern of gene expression was seen from inflammatory acne lesions compared to uninvolved skin from the same patients. Analysis of the gene expression profiles revealed that 211 genes were upregulated at the site of the acne lesion, while 18 genes were down regulated (Refer to supplemental Data). A majority of the genes whose expression is increased in acne lesions were involved with the inflammatory processes, and these included a variety of chemokines, anti-microbial peptides, apoptosis inducing proteins and interstitial collagenases (See Table 1 and Fig1)

Genes with the greatest fold increases in expression in acne lesions included the matrix metalloproteinases MMP-1 and MMP-3, which had 92 and 64-fold higher expression patterns respectively. Other genes significantly upregulated included the pro-inflammatory cytokines IL-8 (52-fold), and CXCL-2 (16-fold). Significant increases in the expression of chemokine receptor 1 (CCR1), IL-7 receptor, IL-13 receptor and IL-1 family members 5&9 were also noted. These data highlight the prominent role of these cytokines in inflammatory acne, which is in agreement with other studies that demonstrate the role of IL-8 in inducing the recruitment of chemotactic mediators at the site of acne lesions (Vowels *et al,* 1995).

The expression of several anti-microbial peptides was also significantly increased in acne lesions compared to normal skin from the same patients. These include HBD-4 and granulysin, which were upregulated by more than 33-fold and 2-fold respectively. Genes involved in apoptosis and immune pathways were also significantly upregulated in acne lesions. These included granzyme B that is responsible for target cell lysis in cell-mediated immune responses and GPR65 that is involved with the differentiation of T-cells as well as their apoptosis. Granzyme B was upregulated more than 10-fold in acne lesions when compared to normal skin while that of GPR65 was increased by approximately 2.7-fold. Tables 1 and 2 provide a more comprehensive list of the genes with statistically significant changes in expression between inflammatory acne lesions and normal skin from the same patients.

While very few genes were downregulated in acne lesions compared to normal skin, some important genes regulating key pathways were identified (Table 2). The frizzled related proteins which functions as part of the 'wnt' signaling pathway were downregulated by approximately 2-fold . Inhibition of wnt signaling in the skin has been shown to influence stem cell fate in favor of development of sebaceous rather than hair (Merrill *et al*, 2001). Also downregulated in acne lesion were three genes of the secretoglobin family: secretoglobin family 1D member2 and secretoglobin family 2A members 1&2. Secretoglobin family 1D member2 and secretoglobin family 2A member1 are transcriptionally regulated by steroids and bind to androgens and other steroids. The function of secretoglobin family 2A member 2 is unclear.

Gene expression profiles from biopsy samples of normal skin from 6 subjects without acne were compared to profiles obtained from normal skin (uninvolved sites) of 6 patients with acne. No significant changes in gene expression patterns were noted in this analysis (data not shown). A subset analysis of target genes was performed and again revealed no significant differences in gene expression between normal skin from subjects with and without acne.

### Example 2: Clustering

Using the computer software dChip (Li and Wong. 2003) we performed hierarchical clustering of the entire set of genes (229) that were significantly upregulated or downregulated from our micro-array data and found that the biopsy samples from inflammatory acne involved skin lesions clustered into a separate group from the uninvolved clinically normal skin from the same group of patients (Refer to supplemental data). Using the NetAffix analysis center from the affymetrix website we identified 41 genes from a total of 211 genes upregulated in inflammatory acne to be involved in inflammation. Figure 1 represents a cluster diagram of the inflammatory genes and shows the unique clustering pattern of the samples into 2 groups, one corresponding to involved skin from acne lesion and another corresponding to clinically un-involved skin form the same group of 6 patients.

### Example 3: qPCR confirms gene array expression data of select genes

We selected 5 genes of interest based on their fold changes and involvement in inflammation from 5 acne subjects to validate the micro-array findings using qPCR. These genes include, MMP-1, MMP-3, IL-8, β defensin 4 and Granzyme B. QPCR results were normalized to the internal control gene, TATA binding protein (TBP). A robust increase in mRNA expression was seen for all 5 genes tested with the magnitude of the fold change greater than that observed with the microarray (Table 3XX Nishit pleaseinclude also the fold changes from microarray in this table).

### Example 4: Immunohistochemistry demonstrates tissue localization of select proteins in inflammatory acne lesions and normal skin.

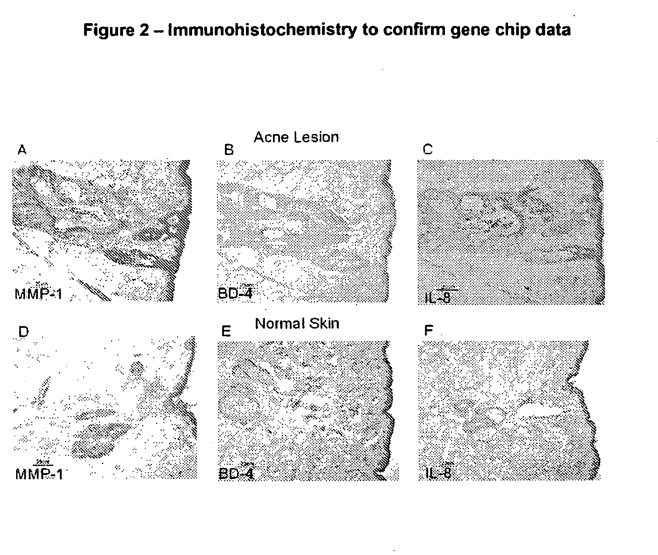

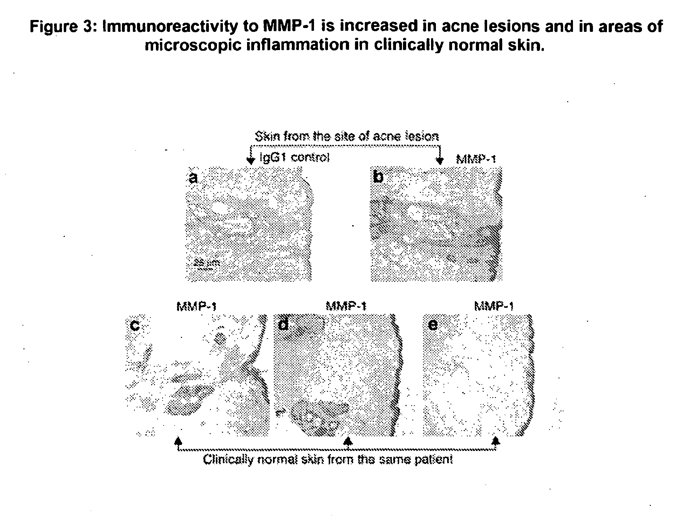

To further confirm the differences in tissue expression and distribution patterns of MMP-1 (interstitial collagenase), IL-8 (pro-inflammatory cytokine) and HBD-4 (an anti-microbial peptide), we performed immunohistochemistry on inflammatory acne lesions, uninvolved skin from acne subjects and on normal skin from subjects without acne. Increased expression of all 3 proteins was noted in inflammatory acne lesions when compared to normal skin from the acne subjects (Figure 2). The expression of HBD-4 was greater in the epidermis of inflammatory acne lesions when compared to the epidermis in the uninvolved skin (Figure 3 A&C). Immunoreactivity with antibody to IL-8 was noted at the follicular and perifollicular sites of the inflammation in the acne lesion (Figure 2B). IL-8 immunoreactivity was relatively absent in the normal skin (Figure 2D).

While we found high levels of MMP-1 expression in the epidermis and sebaceous glands in biopsy sections from the inflammatory acne lesions (fig 3B); we found significant variation in MMP-1 expression in skin biopsies from the uninvolved skin from the same patient (Figure 3C, D, E). Performing serial sectioning of clinically normal skin of a patient with acne we observed perifollicular inflammation, suggestive of an early clinically inapparent acne lesion. MMP-1 immunoreactivity was increased in areas that were close to perifollicular inflammation, while very little MMP-1 immunoreactivity was observed in those areas that had no microscopically visible inflammation. The higher levels of MMP-1 expression at some sites of normal skin need to further examined to determine if changes in MMP-1 expression are amongst the earliest changes prior to the development of comedones and inflammatory acne at that site.

### Discussion

Acne has is recognized as a chronic inflammatory disease, characterized by increased sebum production, abnormal follicular differentiation, and bacterial colonization. Though significant advances have been made in the last decade in identifing the pathophysiological mechanisms involved in acne, there are no published micro-array studies analyzing the differential pattern of gene expression in inflammatory acne lesions and normal skin. Ours is the first study to provide such a comprehensive comparison. In this regard, 211 genes were significantly upregulated in inflammatory acne lesions, many of which, as expected, are involved in inflammation. The major genes whose expression was increased have been implicated in acne. These include matrix metalloproteinases, β-defensin 4, IL-8, and granulysin. In contrast, a much smaller set of genes (18) was downregulated, 3 of which are in the secretoglobin family.

The recognition of microbial pathogens by the cells of the immune system triggers host defense mechanisms to combat infection. These include anti-microbial peptides, inflammatory cytokines and pro-apoptotic enzymes. However, activation of these same mechanisms also results in tissue injury and scarring, a feature commonly observed in inflammatory acne. The data generated in this study supports many of the recent findings regarding inflammatory mediators in acne. For example, the extent to which *P*. *acnes* induces the expression of the antimicrobial peptide, β-defensin 2 (now known as β-defensin 4)) and IL-8 (Nagy, et al., 2005). Since *P*. *acnes* is routinely present in the skin of most individuals, and no correlation between the number of bacteria and the severity and type of acne has been found, it is possible that strain to strain variations in the expression of β-defensin 4 or IL-8 might play a role in the development of inflammatory acne. β-defensin 4 is as an important member of the defensin family of anti-microbial peptides and has strong antimicrobial activity against both gram-positive and gram negative bacteria. Our data demonstrate that β-defensin 4 is expressed in the epidermis of inflammatory acne lesions, but not in normal skin and these results are in agreement with other studies demonstrating an increase in expression of β defensin 1 and 2 in the epidermis of the inflammatory acne lesions (Chronnell, et al., 2001).

Granulysin is another important antimicrobial peptide whose expression is significantly increased in inflammatory acne lesions in our study. In recent years granulysin has gained importance as a peptide that can perform the dual function of being a cytotoxic agent against pathogenic bacteria as well as a pro-inflammatory agent that functions as a chemoattractant and activates monocytes to produce cytokines (Deng, et al., 2005). Antibiotics to combat *P. acnes* and other bacteria have been commonly used in the treatment of acne and thus the upregulation of these anti-microbial peptides produced by the body can be helpful in killing the bacteria. However recent studies have shown that while some chemokines are reduced as a consequence of the microbicidal effect of granulysin peptides, the levels of IL-8 remain unchanged (Mclnturff, et al., 2005).

Interleukin-8, a prototypic human chemokine has been discovered since 1990 as the founding member of the chemokine family. IL-8 is a major mediatory of inflammatory response and a strong chemotactic factor for neutrophis, basophils and T-cells (Zachariae, 1993). Our study demonstrates that it is markedly upregulated in inflammatory acne lesions. The activation of this chemokine is regulated by a combination of 3 major mechanisms namely a) the transcriptional activation by the NF-κB and JUN protein kinase pathways b) stabilization of the mRNA by the p38 MAPK pathway and c) derepression of the gene promotor (Harant *et al,* 1996; Hoffmann *et al,* 2002; Mukaida *et al,* 1994). As in several inflammatory diseases, IL-8 has been implicated to be playing a major role in mounting an inflammatory response in acne lesions. Several studies have focused on the role of I bacteria in inducing IL-8 and discovered that this process may be mediated by the activation of transcription factor NF-kB (Chen *et al*, 2002; Vowels, et al., 1995). Thus a detailed understanding of the pathways governing IL-8 production may help us to identify newer targets and better therapeutics for acne treatment.

The more recent studies by Kang *et al* have focused on identifying the various intra-cellular signaling cascades associated with transcription factors involved in inflammation and matrix degradation in acne lesions. The activation of NF-κB pathway as evidenced by nuclear localization of p65 and p50, and the subsequent regulation of inflammatory cytokines like TNF-α, IL-1β, and IL-8 have provided clues that help our understanding of the molecular pathways governing inflammation in acne (Kang *et al,* 2005). Our results are in agreement with studies by Kang et al. in demonstrating increased expression of matrix metalloproteinases like MMP-1 and MMP-3 and cytokines especially IL-8 in the inflammatory acne lesion. Because MMP-1 expression was also observed in some areas of clinically normal skin, which had microscopically visible areas of inflammation, we hypothesize that MMP-1 could play a role during the initial stages of inflammation and that inflammation may be one of the preceding events to the clinically visible acne lesions as previously suggested (Jeremy, et al., 2003).

Our study has also brought to focus the strong upregulation of granzyme B an essential component of the apoptotic pathway that is necessary for target cell lysis in cell-mediated immune response (Heibein *et al*, 2000). Granzyme B is commonly found in granules produced by cytolytic T lymphocytes (CTLs) and natural killer (NK) cells. CTLs and NK cells use perforin and granzyme B containing granules to destroy cells infected with intracellular pathogens (Trapani and Smyth, 2002). Granzyme B can induce target cell death via two complementary pathways, a cytosolic pathway involving cascade activation of caspases and a nuclear pathway involving CDC-2 activation (Talanian *et al,* 1997). While very little is known about the role played by granzyme B in the pathogenesis of acne, the strong upregulation of granzyme B in acne lesions as observed in our studies will help foster interest into a more in depth study of this protease in acne.

These results strongly bring to light the role of the various proteins involved in inflammation and matrix remodeling in inflammatory acne lesions and provides a more detailed look at the differential patterns of gene expression in an inflammatory acne and clinically normal skin from the same group of patients. The upregulation of antimicrobial peptides is of interest further supporting their role in the endogenous inflammatory response to bacterial pathogens and perhaps also providing a rationale for their potential therapeutic use in acne. Critical questions remain however as to the nature of the initiating events in the development of acne lesions. It is likely that the profiles of gene expression in any inflammatory process in the skin are quite similar and that many of the changes observed in inflammatory lesions are likely to be secondary to as of yet unidentified primary pathogenic events. The challenge lies ahead in identifying these primary events in acne as well as in other inflammatory diseases.

**Table 1 - List of genes up regulated in acne lesions when compared to normal skin**

| **Probe Set ID** | **Accession** | **Fold Change** | **Gene Title** | **Gene Symbol** |
|---|---|---|---|---|
| List of Genes upregulated by more than 10 fold in acne lesions | | | | |
| 204475_at | NM_002421 | 92.166 | matrix melalloproteinase 1 | MMP1 |
| 205828_at | NM_002422 | 64.020 | matrix metalloproteinase 3 | MMP3 |
| 202859_x_at | NM_000584 | 52.521 | interleukin 8 | IL8 |
| 207356_at | NM_004942 | 33.300 | defensin, beta 4 | DEFB4 |
| 203691_at | NM_002638 | 19.354 | protease inhibitor 3, skin-derived (SKALP) | PI3 |
| 41469_at | L10343 | 17.495 | protease inhibitor 3, skin-derived (SKALP) | PI3 |
| 204470_at | NM_001511 | 16.152 | chemokine (C-X-C motif) ligand 2 | CXCL2 |
| 210873_x_at | U03891 | 15.921 | apolipoprotein B mRNA editing enzyme | APOBEC3A |
| 216841_s_at | X15132 | 11.529 | superoxide dismutase 2, mitochondrial | SOD2 |
| 210164_at | J03189 | 10.630 | granzyme B | GZMB |
| | | | | |

| Upregulation of genes involved in the inflammatory pathway (in acne lesions) | | | | |
|---|---|---|---|---|
| 202859_x_at | NM_000584 | 52.521 | interleukin 8 | IL8 |
| 204470_at | NM_001511 | 16.152 | chemokine (C-X-C motif) ligand 2 | CXCL2 |
| 203535_at | NM_002965 | 5.754 | S100 calcium binding protein A9 (calgranulin B) | S100A9 |
| 205098_at | AI421071 | 4.057 | chemokine (C-C motif) receptor 1 | CCR1 |
| 205099_s_at | AI421071 | 3.126 | chemokine (C-C motif) receptor 1 | CCR1 |
| 219403_s_at | AF155510 | 2.920 | heparanase | HPSE |
| 208607_s_at | NM_030754 | 2.659 | serum amyloid A2 | SAA2 |
| 216388_s_at | U33448 | 2.308 | leukotriene B4 receptor | LTB4R |
| 203508_at | NM_001066 | 2.287 | tumor necrosis factor receptor superfamily member 1 B | TNFRSF1B |
| 209906_at | U62027 | 2.238 | complement component 3a receptor 1 | C3AR1 |
| | | | | |

| Upregulation of genes involved in the apoptotic pathway (in acne lesions) | | | | |
|---|---|---|---|---|
| 210164_at | J03189 | 10.630 | granzyme B | GZMB |
| 214467_at | NM_003608 | 2.772 | G protein-coupled receptor 65 | GPR65 |
| 204860_s_at | AI617801 | 2.272 | baculoviral IAP repeat-containing 1 | BIRC1 |
| 201743_at | NM_000591 | 2.211 | CD14 antigen | CD14 |
| 201739_at | NM_005627 | 1.663 | serum/glucocorticoid regulated kinase | SGK |
| 206545_at | NM_006139 | 1.658 | CD28 antigen (Tp44) | CD28 |
| 219366_at | NM_020371 | 1.627 | apoptosis, caspase activation inhibitor | AVEN |
| 206315_x_at | NM_003300 | 1.393 | TNF receptor-associated factor 3 | TRAF3 |
| | | | | |

| Upregulation of genes involved in lipid and steroid metabolism pathway (in acne lesions) | | | | |
|---|---|---|---|---|
| 206561_s_at | NM_020299 | 8.389 | aldo-keto reductase family 1, member B10 | AKR1B10 |
| 203649_s_at | NM_000300 | 5.518 | phospholipase A2, group IIA (platelets, synovial fluid) | PLA2G2A |
| 215125_s_at | AV691323 | 2.804 | UDP glycosyltransferase 1 family, polypeptide A10 | UGT1A10 |
| 202430_s_at | NM_021105 | 2.959 | phospholipid scramblase 1 | PLSCR1 |
| 208607_s_at | NM_030754 | 2.659 | serum amyloid A2 | SAA2 |
| 202345_s_at | NM_001444 | 2.268 | fatty acid binding protein 5 (psoriasis-associated) | FABP5 |
| 204446_s_at | NM_00698 | 2.128 | arachidonate 5-lipoxygenase | ALOX5 |
| 203879_at | U86453 | 1.461 | phosphoinositide-3-kinase, catalytic, delta polypeptide | PIK3CD |

**Table 2: List of genes down regulated in acne lesions compared to normal skin**

| **Probe Set ID** | **Accession** | **Fold Change** | **Gene Title** | **Gene Symbol** |
|---|---|---|---|---|
| | | | | |

| Complete list of genes that were down regulated in acne lesions | | | | |
|---|---|---|---|---|
| 206799_at | NM_006551 | -2.889 | secretoglobin, family 1D, member 2 | SCGB1D2 |
| 205979_at | NM_002407 | -2.883 | secretoglobin, family 2A, member 1 | SCGB2A1 |
| 203824_at | NM_004616 | -2.677 | transmembrane 4 superfamily member 3 | TM4SF3 |
| 212913_at | BE674960 | -2.586 | mutS homolog 5 (E. coli) | MSH5 |
| 206378_at | NM_002411 | -2.227 | secretoglobin, family 2A, member 2 | SCGB2A2 |
| 221406_s_at | NM_025259 | -1.991 | mutS homolog 5 (E. coli) | MSH5 |
| 203697_at | U91903 | -1.939 | frizzled-related protein | FRZB |
| 219127_at | NM_024320 | -1.879 | hypothetical protein MGC11242 | MGC11242 |
| 209842_at | AI367319 | -1.766 | SRY (sex determining region Y)-box 10 | SOX10 |
| 201596_x_at | NM_000224 | -1.656 | keratin 18 | KRT18 |
| 206606_at | NM_000236 | -1.571 | lipase, hepatic | LIPC |
| 205620_at | NM_000504 | -1.547 | coagulation factor X | F10 |
| 219717_at | NM_017741 | -1.522 | hypothetical protein FLJ20280 | FLJ20280 |
| 205388_at | NM_003279 | -1.509 | troponin C2, fast | TNNC2 |
| 207265_s_at | NM_016657 | -1.442 | KDEL endoplasmic reticulum protein retention receptor 3 | KDELR3 |
| 206747_at | NM_146516 | -1.328 | KIAA0514 | KIAA0514 |
| 210006_at | BC002571 | -1.325 | DKFZP5640243 protein | DKFZP5640243 |
| 31637_s_at | X72631 | -1.310 | nuclear receptor subfamily 1, group D, member 1 | NR1D1 |
| | | | | |

**Table 4: list of genes and biomarkers/gene expression products for modulators screening**

| **Accession** | **Gene title** | **Gene symbol** |
|---|---|---|
| NM_004942 | defensin, beta 4 | DEFB4 |
| NM_002638 | protease inhibitor 3, skin-derived (SKALP) | PI3 |
| NM_001511 | chemokine (C-X-C motif) ligand 2 | CXCL2 |
| U03891 | apolipoprotein B mRNA editing enzyme | APOBEC3A |
| X15132 | superoxide dismutase 2, mitochondrial | SOD2 |
| J03189 | granzyme B | GZMB |
| NM_001511 | chemokine (C-X-C motif) ligand 2 | CXCL2 |
| AI421071 | chemokine (C-C motif) receptor 1 | CCR1 |
| U33448 | leukotriene B4 receptor | LTB4R |
| | | |
| NM_001066 | tumor necrosis factor receptor superfamily member 1B | TNFRSF1B |
| U62027 | complement component 3a receptor 1 | C3AR1 |
| NM_003608 | G protein-coupled receptor 65 | GPR65 |
| AI817801 | baculoviral IAP repeat-containing 1 | BIRC1 |
| NM_005627 | serum/glucocorticoid regulated kinase | SGK |
| NM_006139 | CD28 antigen (Tp44) | CD28 |
| NM_020371 | apoptosis, caspase activation inhibitor | AVEN |
| NM_003300 | TNF receptor-associated factor 3 | TRAF3 |
| NM_020299 | aldo-keto reductase family 1, member B10 | AKR1B10 |
| NM_000300 | phospholipase A2, group IIA (platelets, synovial fluid) | PLA2G2A |
| AV691323 | UDP glycosyltransferase 1 family, polypeptide A10 | UGT1A10 |
| NM_021105 | phospholipid scramblase 1 | PLSCR1 |
| NM_030754 | serum amyloid A2 | SAA2 |
| NM_001444 | fatty acid binding protein 5 (psoriasis-associated) | FABP5 |
| U86453 | phosphoinositide-3-kinase, catalytic, delta polypeptide | PIK3CD |
| NM_006551 | secretoglobin, family 1D, member 2 | SCGB1D2 |
| NM_002407 | secretoglobin, family 2A, member 1 | SCGB2A1 |
| NM_004616 | transmembrane 4 superfamily member 3 | TM4SF3 |
| BE674960 | mutS homolog 5 (E. coli) | MSH5 |
| NM_002411 | secretoglobin, family 2A, member 2 | SCGB2A2 |
| U91903 | frizzled-related protein | FRZB |
| AI367319 | SRY (sex determininq region Y)-box 10 | SOX10 |
| NM_000224 | keratin 18 | KRT18 |
| NM_000236 | lipase, hepatic | LIPC |
| NM_000504 | coagulation factor X | F10 |
| NM_003279 | troponin C2, fast | TNNC2 |
| NM_016657 | KDEL endoplasmic reticulum protein retention receptor 3 | KDELR3 |
| X72631 | nuclear receptor subfamily 1, group D, member 1 | NR1D1 |
| Z25521 | CD47 antigen (Rh-related antigen, integrin- associated signal transducer) | CD47 |
| NM_006288 | Thy1 cell surface antigen | Thy1 |
| NM_003006 | selectin P ligand | SELPG |
| NM_003254 | TIMP metallopeptidase inhibitor 1 | TIMP1 |
| NM_002989 | chemokine (C-C motif) ligand 21 | CCL21 |
| NM_002965 | S100 calcium binding protein A9 (calgranulin B) | S100A9 |
| NM_000647 | chemokine (C-C motif) receptor 2 /// chemokine (C-C motif) receptor 2 | CCR2 |
| NM_000579 | chemokine (C-C motif) receptor 5 | CCR5 |
| NM_000655 | selectin L (lymphocyte adhesion molecule 1) | SELL |

### References

Braff MH, Bardan A, Nizet V, Gallo RL: Cutaneous defense mechanisms by antimicrobial peptides. J Invest Dermatol 125: 9-13, 2005.
Chen Q, Koga T, Uchi H, et al: Propionibacterium acnes-induced IL-8 production may be mediated by NF-kappaB activation in human monocytes. J Dermatol Sci 29: 97-103, 2002.
Chronnell CM, Ghali LR, Ali RS, et al: Human beta defensin-1 and -2 expression in human pilosebaceous units: upregulation in acne vulgaris lesions. J Invest Dermatol 117: 1120-5,2001.
. De Young LM, Young JM, Ballaron SJ, Spires DA, Puhvel SM: Intradermal injection of Propionibacterium acnes: a model of inflammation relevant to acne. J Invest Dermatol 83: 394-8, 1984.
Deng A, Chen S, Li Q, Lyu SC, Clayberger C, Krensky AM: Granulysin, a cytolytic molecule, is also a chemoattractant and proinflammatory activator. J Immunol 174: 5243-8, 2005.
Freedberg IM, Tomic-Canic M, Komine M, Blumenberg M: Keratins and the keratinocyte activation cycle. J Invest Dermatol 116: 633-40, 2001.
Harant H, de Martin R, Andrew PJ, Foglar E, Dittrich C, Lindley IJ: Synergistic activation of interleukin-8 gene transcription by all-trans-retinoic acid and tumor necrosis factor-alpha involves the transcription factor NF-kappaB. J Biol Chem 271: 26954-61, 1996.
Harder J, Meyer-Hoffert U, Wehkamp K, Schwichtenberg L, Schroder JM: Differential gene induction of human beta-defensins (hBD-1, -2, -3, and -4) in keratinocytes is inhibited by retinoic acid. J Invest Dermatol 123: 522-9, 2004.
Heibein JA, Goping IS, Barry M, Pinkoski MJ, Shore GC, Green DR, Bleackley RC: Granzyme B-mediated cytochrome c release is regulated by the Bcl-2 family members bid and Bax. J Exp Med 192: 1391-402, 2000.
Hoffmann E, Dittrich-Breiholz O, Holtmann H, Kracht M: Multiple control of interleukin-8 gene expression. J Leukoc Biol 72: 847-55, 2002.
Jappe U, Ingham E, Henwood J, Holland KT: Propionibacterium acnes and inflammation in acne; P. acnes has T-cell mitogenic activity. Br J Dermatol 146: 202-9, 2002.
Jeremy AH, Holland DB, Roberts SG, Thomson KF, Cunliffe WJ: Inflammatory events are involved in acne lesion initiation. J Invest Dermatol 121: 20-7, 2003.
Kang S, Cho S, Chung JH, Hammerberg C, Fisher GJ, Voorhees JJ: Inflammation and extracellular matrix degradation mediated by activated transcription factors nuclear factor-kappaB and activator protein-1 in inflammatory acne lesions in vivo. Am J Pathol 166: 1691-9, 2005.
Kim J, Ochoa MT, Krutzik SR, et al: Activation of toll-like receptor 2 in acne triggers inflammatory cytokine responses. J Immunol 169: 1535-41, 2002.
Mclnturff JE, Wang SJ, Machleidt T, et al: Granulysin-derived peptides demonstrate antimicrobial and anti-inflammatory effects against Propionibacterium acnes. J Invest Dermatol 125: 256-63, 2005.
Merrill BJ, Gat U, DasGupta R, Fuchs E: Tcf3 and Lef1 regulate lineage differentiation of multipotent stem cells in skin. Genes Dev 15: 1688-705, 2001.
Mukaida N, Okamoto S, Ishikawa Y, Matsushima K: Molecular mechanism of interleukin-8 gene expression. J Leukoc Biol 56: 554-8, 1994.
Nagy I, Pivarcsi A, Koreck A, Szell M, Urban E, Kemeny L: Distinct strains of Propionibacterium acnes induce selective human beta-defensin-2 and interleukin-8 expression in human keratinocytes through toll-like receptors. J Invest Dermatol 124: 931-8, 2005.
Schaller M, Loewenstein M, Borelli C, Jacob K, Vogeser M, Burgdorf WH, Plewig G: Induction of a chemoattractive proinflammatory cytokine response after stimulation of keratinocytes with Propionibacterium acnes and coproporphyrin III. Br J Dermatol 153: 66-71, 2005.
Schroder JM: [Epithelial antimicrobial peptides: local innate defense effector molecules]. Ann Dermatol Venereol 131: 411-6, 2004.
Selsted ME, Ouellette AJ: Mammalian defensins in the antimicrobial immune response. Nat Immunol 6: 551-7, 2005.
Talanian RV, Yang X, Turbov J, et al: Granule-mediated killing: pathways for granzyme B-initiated apoptosis. J Exp Med 186: 1323-31, 1997.
Trapani JA, Smyth MJ: Functional significance of the perforin/granzyme cell death pathway. Nat Rev Immunol 2: 735-47, 2002.
Vowels BR, Yang S, Leyden JJ: Induction of proinflammatory cytokines by a soluble factor of Propionibacterium acnes: implications for chronic inflammatory acne. Infect Immun 63: 3158-65, 1995.
. Zachariae CO: Chemotactic cytokines and inflammation. Biological properties of the lymphocyte and monocyte chemotactic factors ELCF, MCAF and IL-8. Acta Derm Venereol Suppl (Stockh) 181: 1-37, 1993.

## Claims

1. Acne diagnostic method or an acne disease or hyperseborrhoea evolution follow-up method by using expression of genes or biomarkers/ gene expression products selected from the following list: receptor 1 chemokine (C-C motif) (CCR1); chemokine (C-C motif) receptor 2 /// chemokine (C-C motif) receptor 2 (CCR2); chemokine (C-C motif) receptor 5 (CCR5)said method comprising the step of comparing expression of said genes, for genes expression products activity, in a patient biological sample with a subject "control" biological sample.

2. Acne diagnostic method according to claim 1 **characterized in that** acne comprises simple acne, comedonic acne, papulopustular acne, papulocomedonic acne, nodulocystic acne, acne conglobata, cheloid acne of the nape of the neck, recurrent miliary acne, necrotic acne, neonatal acne, occupational acne, acne rosacea, senile acne, solar acne and medication-related acne.

3. In vitro screening method of drug candidate susceptible of preventing and/or treating acne as well as hyperseborrehoea, comprising determining the capacity of said candidate to inhibit or down regulate expression of a selected gene or activity of said selected gene expression product selected from the following list: receptor 1 chemokine (C-C motif) (CCR1); chemokine (C-C motif) receptor 2 /// chemokine (C-C motif) receptor 2 (CCR2); chemokine (C-C motif) receptor 5 (CCR5).

4. In vitro screening method of drug candidates according to claim 3, comprising the following steps:
a) Collecting at least two biological samples: one mimics the acne lesion, and one mimics the healthy condition;
b) Contacting at least one sample or a mixture of samples with one or more drug candidates to be tested;
c) Measuring gene expression or gene expression product level or activity in the biological samples or mixture obtained in b); the said gene expression product is selected from the following list: receptor 1 chemokine (C-C motif) (CCR1); chemokine (C-C motif) receptor 2 /// chemokine (C-C motif) receptor 2 (CCR2); chemokine (C-C motif) receptor 5 (CCR5);
d) Selecting drug candidates which are capable of modulating gene expression or gene expression product level or activity measured in said samples or mixture obtained in b) and comparing the levels with a sample not mixed with the drug candidate.

5. Method according to claim 4, wherein the drug candidates at step d) are inhibitors of up-regulated gene expression product which is selected from the following list:receptor 1 chemokine (C-C motif) (CCR1); chemokine (C-C motif) receptor 2 /// chemokine (C-C motif) receptor 2 (CCR2); chemokine (C-C motif) receptor 5 (CCR5).

6. Method according to claims 3 to 5 **characterized in that** acne comprises simple acne, comedonic acne, papulopustular acne, papulacomedonic acne, nodulocystic acne, acne conglobata, cheloid acne of the nape of the neck, recurrent miliary acne, necrotic acne, neonatal acne, occupational acne, acne rosacea, senile acne, solar acne and medication-related acne

7. In vitro determination method of patient sensitivity to develop acne lesions and/or hyperseborrhoea, which comprises the step of comparing genes expression or gene expression products levels, or activity of biomarkers as selected from the following list: chemokine (C-C motif) receptor 1 (CCR1); chemokine (C-C motif) receptor 2 /// chemokine (C-C motif) receptor 2 (CCR2); chemokine (C-C motif) receptor 5 (CCR5), in a patient biological sample with a subject "control" biological sample.

8. In vitro determination method according to claim 7 **characterized in that** acne comprises simple acne, comedonic acne, papulopustular acne, papulocomedonic acne, nodulocystic acne, acne conglobata, cheloid acne of the nape of the neck, recurrent miliary acne, necrotic acne, neonatal acne, occupational acne, acne rosacea, senile acne, solar acne and medication-related acne

## Patentansprüche

1. Aknediagnostikverfahren oder ein Akneerkrankungs- oder Hyperseborrhoeentwicklungs-Verlaufskontrollverfahren durch Verwenden der Expression von Genen oder Biomarkern/Genexpressionsprodukten ausgewählt aus der folgenden Liste: Rezeptor-1-Chemokin (C-C Motiv) (CCR1); Chemokin-(C-C Motiv)-Rezeptor 2 /// Chemokin-(C-C Motiv)-Rezeptor 2 (CCR2); Chemokin-(C-C Motiv)-Rezeptor 5 (CCR5), wobei das Verfahren den Schritt umfasst des Vergleichens der Expression der Gene oder der Aktivität der Genexpressionsprodukte in einer biologischen Probe des Patienten mit einer Subjekt-"Kontroll"-biologischen Probe.

2. Aknediagnostikverfahren nach Anspruch 1, **dadurch gekennzeichnet, dass** Akne Acne simplex, Acne comedonica, Acne papulopustulosa, Acne papulocomedonica, nodulocystische Akne, Acne conglobata, keloide Akne am Nacken, wiederkehrende Acne miliaris, nekrotische Akne, Acne neonatorum, berufsbedingte Akne, Acne rosacea, senile Akne, Acne solaris und Medikamenten-bedingte Akne umfasst.

3. In vitro Durchsuchungsverfahren eines Kandidatenarzneimittels empfänglich Akne sowie Hyperseborrhoe zu verhindern und/oder zu behandeln, umfassend Bestimmen der Fähigkeit des Kandidaten, die Expression eines ausgewählten Gens oder die Aktivität des ausgewählten Genexpressionsprodukts ausgewählt aus der folgenden Liste: Rezeptor-1-Chemokin (C-C Motiv) (CCR1); Chemokin-(C-C Motiv)-Rezeptor 2 /// Chemokin-(C-C Motiv)-Rezeptor 2 (CCR2); Chemokin-(C-C Motiv)-Rezeptor 5 (CCR5) zu inhibieren oder herunter zu regulieren.

4. In vitro Durchsuchungsverfahren eines Kandidatenarzneimittels nach Anspruch 3, umfassend die folgenden Schritte:
a) Sammeln von mindestens zwei biologischen Proben: eine imitiert die Akneverletzung, und eine imitiert den gesunden Zustand;
b) Kontaktieren mindestens einer Probe oder eine Mischung von Proben mit einem oder mehreren Arzneimittelkandidaten, die getestet werden sollen;
c) Messen der Genexpression oder des Genexpressionsproduktslevels oder -aktivität in den biologischen Proben oder Mischung erhalten in Schritt b); das Genexpressionprodukt ist ausgewählt aus der folgenden Liste: Rezeptor-1-Chemokin (C-C Motiv) (CCR1); Chemokin-(C-C Motiv)-Rezeptor 2 /// Chemokin-(C-C Motiv)-Rezeptor 2 (CCR2); Chemokin-(C-C Motiv)-Rezeptor 5 (CCR5);
d) Auswählen der Arzneimittelkandidaten, die fähig sind, die Genexpression oder den Genexpressionproduktlevel oder -aktivität gemessen in den Proben oder Mischung erhalten in b) zu modulieren und vergleichen der Level mit einer Probe, die nicht mit dem Kandidatenarzneimittel gemischt wurde.

5. Verfahren nach Anspruch 4, wobei die Kandidatenarzneimittel in Schritt d) Inhibitoren des hochregulierten Genexpressionsprodukts sind, das ausgewählt ist aus der folgenden Liste: Rezeptor-1-Chemokin (C-C Motiv) (CCR1); Chemokin-(C-C Motiv)-Rezeptor 2 /// Chemokin-(C-C Motiv)-Rezeptor 2 (CCR2); Chemokin-(C-C Motiv)-Rezeptor 5 (CCR5).

6. Verfahren nach den Ansprüchen 3 bis 5, **dadurch gekennzeichnet, dass** Akne Acne simplex, Acne comedonica, Acne papulopustulosa, Acne papulocomedonica, nodulocystische Akne, Acne conglobata, keloide Akne am Nacken, wiederkehrende Acne miliaris, nekrotische Akne, Acne neonatorum, berufsbedingte Akne, Acne rosacea, senile Akne, Acne solaris und Medikamenten-bedingte Akne umfasst.

7. In vitro Bestimmungsverfahren der Sensitivität eines Patienten, Akneverletzungen und/oder Hyperseborrhoe zu entwickeln, welche den Schritt umfasst des Vergleichens der Genexpression oder der Genexpressionsproduktlevel oder Aktivität der Biomarker wie ausgewählt aus der folgenden Liste: Chemokin-(C-C Motiv)-Rezeptor 1 (CCR1); Chemokin-(C-C Motiv)-Rezeptor 2 /// Chemokin-(C-C Motiv)-Rezeptor 2 (CCR2); Chemokin-(C-C Motiv)-Rezeptor 5 (CCR5), in einer biologischen Probe eines Patienten mit einer Subjekt-"Kontroll"-biologischen Probe.

8. In vitro Bestimmungsverfahren nach Anspruch 7, **dadurch gekennzeichnet, dass** Akne Acne simplex, Acne comedonica, Acne papulopustulosa, Acne papulocomedonica, nodulocystische Akne, Acne conglobata, keloide Akne am Nacken, wiederkehrende Acne miliaris, nekrotische Akne, Acne neonatorum, berufsbedingte Akne, Acne rosacea, senile Akne, Acne solaris und Medikamenten-bedingte Akne umfasst.

## Revendications

1. Méthode de diagnostic de l'acné ou méthode de suivi de l'évolution d'une maladie acnéique ou d'une hyperséborrhée par utilisation de l'expression de gènes ou de biomarqueurs/ produits d'expression de gènes choisis dans la liste suivante : récepteur 1 de chimiokine (motif C-C) (CCR1) ; récepteur 2 de chimiokine (motif C-C) /// récepteur 2 de chimiokine (motif C-C) (CCR2) ; récepteur 5 de chimiokine (motif C-C) (CCR5), ladite méthode comprenant l'étape de comparaison de l'expression desdits gènes, ou de l'activité des produits d'expression des gènes, dans un échantillon biologique de patient et dans un échantillon biologique d'un sujet « contrôle ».

2. Méthode de diagnostic de l'acné selon la revendication 1 **caractérisée en ce que** l'acné comprend l'acné simple, l'acné comédonienne, l'acné papulopustulaire, l'acné papulocomédonienne, l'acné nodulokystique, l'acné conglobata, l'acné chéloïde de la nuque, l'acné miliaire récurrente, l'acné nécrotique, l'acné néonatale, l'acné professionnelle, l'acné rosacée, l'acné sénile, l'acné solaire et l'acné médicamenteuse.

3. Méthode de criblage in vitro d'un candidat médicament susceptible de prévenir et/ou traiter aussi bien l'acné que l'hyperséborrhée, comprenant la détermination de la capacité dudit candidat à inhiber ou à diminuer l'expression d'un gène sélectionné ou l'activité du produit d'expression dudit gène sélectionné choisi dans la liste suivante : récepteur 1 de chimiokine (motif C-C) (CCR1) ; récepteur 2 de chimiokine (motif C-C) /// récepteur 2 de chimiokine (motif C-C) (CCR2) ; récepteur 5 de chimiokine (motif C-C) (CCR5).

4. Méthode de criblage in vitro d'un candidat médicament selon la revendication 3, comprenant les étapes suivantes :
a) Prélèvement d'au moins deux échantillons biologiques : l'un représente l'état de lésion acnéique, et l'autre représente l'état sain ;
b) Mise en contact d'au moins un échantillon ou d'un mélange d'échantillons avec un ou plusieurs candidats médicaments à tester ;
c) Mesure de l'expression du gène ou du niveau ou de l'activité du produit d'expression du gène dans les échantillons biologiques ou le mélange obtenus en b) ; ledit produit d'expression du gène est choisi dans la liste suivante : récepteur 1 de chimiokine (motif C-C) (CCR1) ; récepteur 2 de chimiokine (motif C-C) /// récepteur 2 de chimiokine (motif C-C) (CCR2) ; récepteur 5 de chimiokine (motif C-C) (CCR5) ;
d) Sélection des candidats médicaments qui sont capables de moduler l'expression du gène ou le niveau ou l'activité du produit d'expression du gène mesuré dans lesdits échantillons ou mélange obtenus en b) et comparaison des niveaux avec un échantillon non mélangé avec le candidat médicament.

5. Méthode selon la revendication 4, dans laquelle les candidats médicaments à l'étape d) sont des inhibiteurs d'un produit d'expression de gène régulé à la hausse qui est choisi dans la liste suivante : récepteur 1 de chimiokine (motif C-C) (CCR1) ; récepteur 2 de chimiokine (motif C-C) /// récepteur 2 de chimiokine (motif C-C) (CCR2) ; récepteur 5 de chimiokine (motif C-C) (CCR5).

6. Méthode selon les revendications 3 à 5 **caractérisée en ce que** l'acné comprend l'acné simple, l'acné comédonienne, l'acné papulopustulaire, l'acné papulocomédonienne, l'acné nodulokystique, l'acné conglobata, l'acné chéloïde de la nuque, l'acné miliaire récurrente, l'acné nécrotique, l'acné néonatale, l'acné professionnelle, l'acné rosacée, l'acné sénile, l'acné solaire et l'acné médicamenteuse.

7. Méthode de détermination in vitro de la sensibilité d'un patient à développer des lésions d'acné et/ou une hyperséborrhée, qui comprend l'étape de comparaison de l'expression des gènes ou des niveaux des produits d'expression de gène, ou de l'activité des biomarqueurs tels que choisis dans la liste suivante : récepteur 1 de chimiokine (motif C-C) (CCR1) ; récepteur 2 de chimiokine (motif C-C) /// récepteur 2 de chimiokine (motif C-C) (CCR2) ; récepteur 5 de chimiokine (motif C-C) (CCR5), dans un échantillon biologique de patient avec un échantillon biologique d'un sujet « contrôle ».

8. Méthode de détermination in vitro selon la revendication 7 **caractérisée en ce que** l'acné comprend l'acné simple, l'acné comédonienne, l'acné papulopustulaire, l'acné papulocomédonienne, l'acné nodulokystique, l'acné conglobata, l'acné chéloïde de la nuque, l'acné miliaire récurrente, l'acné nécrotique, l'acné néonatale, l'acné professionnelle, l'acné rosacée, l'acné sénile, l'acné solaire et l'acné médicamenteuse.
